# EUROPEAN PATENT APPLICATION

(11) **EP 4 545 060 A1**
(43) Date of publication of application: **30.04.2025**
(21) Application number: 23827024.3
(22) Date of filing: 09.06.2023
(51) Int. Cl.: A61K 6/887, A61C 5/70, A61C 13/087, A61C 13/15, A61K 6/836

(54) **COMPOSITION FOR THREE-DIMENSIONAL MODELING AND METHOD FOR PRODUCING DENTAL MODELED OBJECT**

(30) Priority: 21.06.2022 JP 2022099509
(71) Applicant: GC Corporation, Sunto-gun, Shizuoka 410-1307 (JP)
(72) Inventor: KAKINUMA, Hiroaki, Tokyo 174-8585 (JP); TAKADA, Daisuke, Tokyo 174-8585 (JP); KATO, Hiroki, Tokyo 174-8585 (JP); UENO, Takayuki, Tokyo 174-8585 (JP)
(74) Representative: Müller-Boré & Partner Patentanwälte PartG mbB
(86) International application number: PCT/JP2023/021605
(87) International publication number: WO 2023/248838

(57) **Abstract**

A three-dimensional printing composition includes: a polymerizable monomer; inorganic particles; and a photopolymerization initiator. The inorganic particles are surface-treated with a compound represented by a general formula (1) 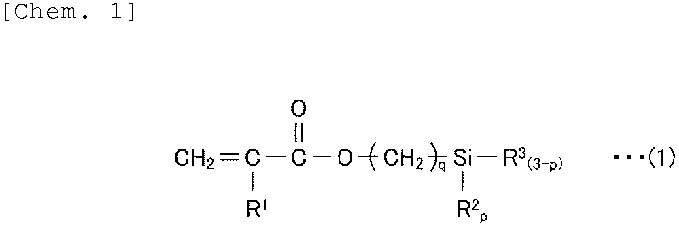 (in the general formula, R1 is a hydrogen atom or a methyl group; R2 is a hydrolyzable group; R3 is a hydrocarbon group having 1 or more and 6 or less carbon atoms; p is 2 or 3; and q is an integer of 5 or more and 13 or less).

## Description

### Technical Field

The present invention relates to a three-dimensional printing composition and a method of producing a dental printed article.

### Background Art

In recent years, technology for three-dimensional printing of articles has developed, and for example, in the field of dentistry, technology for three-dimensional printing of dental printed articles is known (see Patent Documents 1 and 2).

### Citation List

### Patent Document

Patent Document 1: Japanese Unexamined Patent Application Publication No. 2015-43793
Patent Document 2: Japanese Translation of PCT International Application Publication No. JP-T-2016-505525

### Summary of Invention

### Technical Problem

A three-dimensional printed article is required to have high abrasion resistance and mechanical strength, while being able to be three-dimensionally printed.

An object of the present invention is to provide a three-dimensional printing composition that can provide a three-dimensional printed article having high abrasion resistance and mechanical strength.

### Solution to Problem

According to one aspect of the present invention, a three-dimensional printing composition includes: a polymerizable monomer; inorganic particles; and a photopolymerization initiator. The inorganic particles are surface-treated with a compound represented by a general formula (1) below. 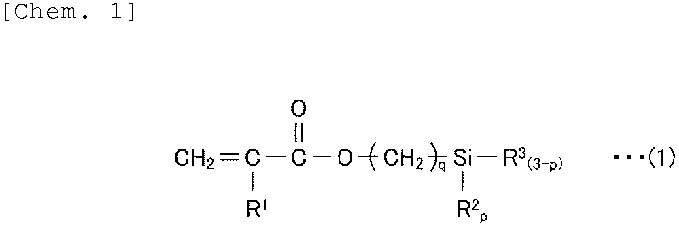 (in the general formula, R1 is a hydrogen atom or a methyl group; R2 is a hydrolyzable group; R3 is a hydrocarbon group having 1 or more and 6 or less carbon atoms; p is 2 or 3; and q is an integer of 5 or more and 13 or less).

### Advantageous Effects of Invention

According to one aspect of the present invention, it is possible to provide a three-dimensional printing composition that can provide a three-dimensional printed article having high abrasion resistance and mechanical strength.

### Description of Embodiments

In the following, embodiments of the present invention will be described.

### <Three-Dimensional Printing Composition>

A three-dimensional printing composition according to the present embodiment includes a polymerizable monomer, inorganic particles, and a photopolymerization initiator.

### [Polymerizable Monomer]

The polymerizable monomer is not particularly limited, but is, for example, a (meth)acrylate. As used herein, the (meth)acrylate refers to an acrylate and/or a methacrylate.

Examples of the (meth)acrylate include ethoxylated bisphenol A dimethacrylate, triethylene glycol dimethacrylate, urethane dimethacrylate, neopentyl glycol dimethacrylate, glycerol dimethacrylate, and the like. Among these, (meth)acrylates having no urethane group, such as ethoxylated bisphenol A dimethacrylate and triethylene glycol dimethacrylate, are preferable. Among these (meth)acrylates, one type may be used alone or two or more types may be used in combination.

The content of the polymerizable monomer in the three-dimensional printing composition is not particularly limited, but for example, the lower limit is preferably 40 mass% or more, more preferably 45 mass% or more, and even more preferably 50 mass% or more. The upper limit is, for example, preferably 80 mass% or less, more preferably 75 mass% or less, and even more preferably 70 mass% or less.

When the content of the polymerizable monomer is 40 mass% or more, the viscosity of the three-dimensional printing composition can be decreased, and the bending strength of the three-dimensional printed article can be improved. When the content of the polymerizable monomer is 80 mass% or less, the abrasion resistance and the bending strength of the obtained three-dimensional printed article can be improved.

### [Inorganic Particles]

The inorganic particles are surface-treated with a compound represented by the following general formula (1): 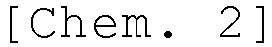 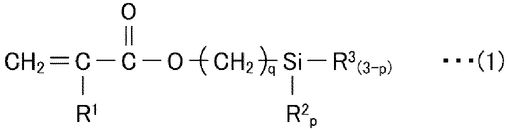 In the formula (1), R1 is a hydrogen atom or a methyl group; R2 is a hydrolyzable group; R3 is a hydrocarbon group having 1 or more and 6 or less carbon atoms; p is 2 or 3; and q is an integer of 5 or more and 13 or less. q is preferably an integer of 6 or more and 11 or less, and more preferably an integer of 7 or more and 9 or less.

The compound for surface treatment of the inorganic particles is not particularly limited, but is, for example, a surface treating agent such as 5-methacryloyloxypentyltrimethoxysilane, 6-methacryloyloxyhexyltrimethoxysilane, 7-methacryloyloxyheptyltrimethoxysilane, 8-methacryloyloxyoctyltrimethoxysilane, 9-methacryloyloxynonyltrimethoxysilane, 10-methacryloyloxydecyltrimethoxysilane, 11-methacryloyloxyundecyltrimethoxysilane, 12-methacryloyloxydodecyltrimethoxysilane, 13-methacryloyloxytridecyltrimethoxysilane, 5-methacryloyloxypentyltriethoxysilane, 6-methacryloyloxyhexyltriethoxysilane, 7-methacryloyloxyheptyltriethoxysilane, 8-methacryloyloxyoctyltriethoxysilane, 9-methacryloyloxynonyltriethoxysilane, 10-methacryloyloxydecyltriethoxysilane, 11-methacryloyloxyundecyltriethoxysilane, 12-methacryloyloxydodecyltriethoxysilane, 13-methacryloyloxytridecyltriethoxysilane, 5-methacryloyloxypentyltripropoxysilane, 6-methacryloyloxyhexyltripropoxysilane, 7-methacryloyloxyheptyltripropoxysilane, 8-methacryloyloxyoctyltripropoxysilane, 9-methacryloyloxynonyltripropoxysilane, 10-methacryloyloxydecyltripropoxysilane, 11-methacryloyloxyundecyltripropoxysilane, 12-methacryloyloxydodecyltripropoxysilane, 13-methacryloyloxytridecyltripropoxysilane, and the like. Among these surface treating agents, one type may be used alone or two or more types may be used in combination.

Examples of the inorganic particles to be surface-treated include various kinds of glasses (for example, E-glass, barium glass, lanthanum glass), various kinds of ceramics, complex oxides (for example, silica-titania complex oxide, silica-zirconia complex oxide), kaolin, clay minerals (for example, montmorillonite), mica, ytterbium fluoride, yttrium fluoride, and the like, that are mainly composed of silica and optionally contain oxides of heavy metals, boron, aluminum, and the like. Among these inorganic particles, one type may be used alone or two or more types may be used in combination.

A volume median particle diameter of the inorganic particles is preferably 0.1 µm or more and 2.0 µm or less, more preferably 0.2 µm or more and 1.5 µm or less, and even more preferably 0.3 µm or more and 1.0 µm or less. When the volume median particle diameter of the inorganic particles is 0.1 µm or more, a decrease in bending strength of the cured body of the three-dimensional printing composition is minimized, and when the volume median particle diameter is 2.0 µm or less, a decrease in abrasion resistance is minimized.

In the present specification, the volume median particle diameter refers to the median particle diameter in the volume particle size distribution. The volume particle size distribution may be measured by a laser diffraction/scattering method.

The content of the inorganic particles in the three-dimensional printing composition is not particularly limited, but for example, the lower limit is preferably 30 mass% or more, more preferably 35 mass% or more, and even more preferably 40 mass% or more. The upper limit is, for example, preferably 60 mass% or less, more preferably 55 mass% or less, and even more preferably 50 mass% or less.

When the content of the inorganic particles is 30 mass% or more, the abrasion resistance and bending strength of the printed article of the three-dimensional printing composition can be improved. When the content of the inorganic particles is 60 mass% or less, an increase in the viscosity of the three-dimensional printing composition is minimized, and the production of the three-dimensional printed article is facilitated.

### [Photopolymerization Initiator]

The photopolymerization initiator is not particularly limited, but is, for example, camphorquinone, benzyl, diacetyl, benzyldimethylketal, benzyldiethylketal, benzylbis (2-methoxyethyl) ketal, 4,4'-dimethylbenzyl-dimethylketal, anthraquinone, 1-chloroanthraquinone, 2-chloroanthraquinone, 1,2-benzanthraquinone, 1-hydroxyanthraquinone, 1-methylanthraquinone, 2-ethylanthraquinone, 1-bromoanthraquinone, thioxanthone, 2-isopropylthioxanthone, 2-nitrothioxanthone, 2-methylthioxanthone, 2,4-dimethylthioxanthone, 2,4-diethylthioxanthone, 2,4-diisopropylthioxanthone, 2-chloro-7-trifluoromethylthioxanthone, thioxanthone-10,10-dioxide, thioxanthone-10-oxide, benzoin methyl ether, benzoin ethyl ether, isopropyl ether, benzoin isobutyl ether, benzophenone, bis(4-dimethylaminophenyl) ketone, 4,4'-bisdiethylaminobenzophenone, 2,4,6-trimethylbenzoyldiphenylphosphine oxide, phenylbis(2,4,6-trimethylbenzoyl) phosphine oxide, and the like. Among these photopolymerization initiators, one type may be used alone or two or more types may be used in combination.

The content of the photopolymerization initiator in the three-dimensional printing composition is not particularly limited, but for example, the lower limit is preferably 0.01 mass% or more, more preferably 0.05 mass% or more, and even more preferably 0.1 mass% or more. The upper limit is, for example, preferably 10 mass% or less, more preferably 5 mass% or less, and even more preferably 1 mass% or less.

When the content of the photopolymerization initiator is 0.01 mass% or more, the production of the three-dimensional printed article is facilitated. When the content of the polymerization initiator is 10 mass% or less, the production of the three-dimensional printed article is facilitated, and discoloration of the printed article is minimized.

The three-dimensional printing composition according to the present embodiment may further include a tertiary amine, a polymerization inhibitor, an ultraviolet absorber, a pigment, or the like, as required.

Examples of the tertiary amine include a tertiary aliphatic amine such as N,N-dimethylaminoethyl methacrylate, triethanolamine, or the like; a tertiary aromatic amine such as alkyl p-dialkylaminobenzoate (for example, methyl p-dimethylaminobenzoate, ethyl p-dimethylaminobenzoate, propyl p-dimethylaminobenzoate, amyl p-dimethylaminobenzoate, isoamyl p-dimethylaminobenzoate, ethyl p-diethylaminobenzoate, propyl p-diethylaminobenzoate, and the like), 7-dimethylamino-4 methylcoumarin, N,N-dimethylaniline, N,N-dibenzylaniline, N,N-dimethyl-p-toluidine, N,N-diethyl-p-toluidine, N,N-bis(2-hydroxyethyl)-p-toluidine, N,N,2,4,6-pentamethylaniline, N,N,2,4-tetramethylaniline, N,N-diethyl-2,4,6-trimethylaniline, or the like; and the like.

Among these tertiary amines, one type may be used alone or two or more types may be used in combination. Among these, a tertiary aromatic amine is preferable, and an alkyl p-dialkylaminobenzoate is more preferable.

The content of the tertiary amine in the three-dimensional printing composition is not particularly limited, but is preferably 0.01 mass% or more and 10 mass% or less, more preferably 0.05 mass% or more and 5 mass% or less, and even more preferably 0.1 mass% or more and 1 mass% or less. When the content of the tertiary amine in the three-dimensional printing composition is 0.01 mass% or more and 10 mass% or less, the abrasion resistance and mechanical strength of the three-dimensional printing composition are improved.

Examples of the polymerization inhibitor include 2,6-di-tert-butyl-p-cresol, 6-tert-butyl-2,4-xylenol, hydroquinone, dibutylhydroquinone, dibutylhydroquinone monomethyl ether, 2,6-di-tertbutylphenol, 4-methoxyphenol, and the like. Among these polymerization inhibitors, one type may be used alone or two or more types may be used in combination.

The content of the polymerization inhibitor in the three-dimensional printing composition is not particularly limited, but is preferably 0.0005 mass% or more and 5 mass% or less, more preferably 0.001 mass% or more and 3 mass% or less, and even more preferably 0.005 mass% or more and 1 mass% or less. When the content of the photopolymerization initiator in the three-dimensional printing composition is 0.0005 mass% or more and 5 mass% or less, the storage stability of the three-dimensional printing composition is improved.

Examples of the ultraviolet absorber include 2,5-bis(5-tert-butyl-2-benzoxazolyl) thiophene, 2-(2H-benzotriazole-2-yl)-4-methylphenol, 2-ethylhexyl 2-cyano-3,3-diphenylacrylate, and the like.

Among the ultraviolet absorbers, one type may be used alone or two or more types may be used in combination. Among these, 2,5-bis(5-tert-butyl-2-benzoxazolyl) thiophene is preferable from the viewpoint of improving the printability of the obtained three-dimensional printed article, and 2-(2H-benzotriazole-2-yl)-4-methylphenol, 2-ethylhexyl 2-cyano-3,3-diphenylacrylate, and the like are preferable from the viewpoint of improving the photostability of the three-dimensional printing composition.

The content of the ultraviolet absorber in the three-dimensional printing composition is not particularly limited, but is preferably 0.0005 mass% or more and 5 mass% or less, more preferably 0.001 mass% or more and 3 mass% or less, and even more preferably 0.005 mass% or more and 1 mass% or less. When the content of the photopolymerization initiator in the three-dimensional printing composition is 0.0005 mass% or more and 5 mass% or less, the storage stability of the three-dimensional printing composition is improved, and the printability of the obtained three-dimensional printed article is improved.

Examples of the pigment include titanium dioxide (white), iron oxide (yellow/red), triiron tetroxide (black), and the like. Among these pigments, one type may be used alone or two or more types may be used in combination.

The content of the pigment in the three-dimensional printing composition is preferably 0.001 mass% or more and 1 mass% or less, and more preferably 0.05 mass% or more and 0.5 mass% or less.

The viscosity of the three-dimensional printing composition according to the present embodiment is, for example, preferably 200 mPa·s or more and 1,500 mPa·s or less, more preferably 300 mPa·s or more and 1,300 mPa·s or less, and even more preferably 400 mPa·s or more and 1,000 mPa·s or less. When the viscosity of the three-dimensional printing composition is 200 mPa·s or more and 1,500 mPa·s or less, the three-dimensional printing composition can be easily printed. Here, the viscosity refers to the viscosity at 35°C measured by a type B viscometer.

As described above, the three-dimensional printing composition according to the present embodiment includes the polymerizable monomer, the inorganic particles, and the photopolymerization initiator, and the inorganic particles are surface-treated with the compound represented by the above general formula (1). Thus, the three-dimensional printing composition according to the present embodiment can improve abrasion resistance and mechanical strength of the obtained three-dimensional printed article while enabling three-dimensional printing.

Further, as described above, in the three-dimensional printing composition according to the present embodiment, the volume median particle diameter of the inorganic particles is 0.1 µm or more and 2.0 µm or less. Therefore, the dental printed article obtained by the three-dimensional printing using such a three-dimensional printing composition can maintain high abrasion resistance and mechanical strength.

Further, as described above, the viscosity of the three-dimensional printing composition according to the present embodiment is 200 mPa·s or more and 1,500 mPa·s or less. Therefore, the dental printed article obtained by the three-dimensional printing using such a three-dimensional printing composition can be easily obtained by the three-dimensional printing.

The use of the three-dimensional printing composition according to the present embodiment is not limited, but it can be used, for example, for three-dimensional printing of a dental printed article.

The dental printed article to be three-dimensionally printed is not particularly limited, but includes, for example, dental prostheses such as crowns, bridges, inlays, onlays, veneers, temporary dentures, artificial teeth, and the like.

When the three-dimensional printing composition according to the present embodiment is used for three-dimensional printing of the dental printed article, the effect obtained by the three-dimensional printing composition according to the present embodiment can be obtained as well in the obtained dental printed article.

Specifically, as described above, the three-dimensional printing composition according to the present embodiment includes the polymerizable monomer, the inorganic particles, and the photopolymerization initiator, and the inorganic particles are surface-treated with the compound represented by the above general formula (1). Therefore, the dental printed article obtained by the three-dimensional printing using such a three-dimensional printing composition can exhibit high abrasion resistance and mechanical strength.

### <Method of Producing Dental Printed article>

A method of producing a dental printed article according to the present embodiment includes a step of laminating the three-dimensional printing composition according to the present embodiment, thereby three-dimensionally printing a dental printed article. The dental printed article obtained by the production method according to the present embodiment is not particularly limited, but is, for example, the dental prosthesis to be three-dimensionally printed using the three-dimensional printing composition described above.

A known 3D printer can be used for laminating the three-dimensional printing composition. Examples of the method used by the 3D printer include a stereolithography (SLA) method, a digital light processing (DLP) method, and the like, and the DLP method is preferable. Commercially available 3D printers employing the DLP method include, for example, MAX UV (manufactured by Asiga).

Examples of the method of laminating the three-dimensional printing composition include a method of irradiating a vat containing the three-dimensional printing composition with light from above (a free liquid level method), a method of irradiating a vat with light from below (a constrained liquid level method), and the like. Among these, the constrained liquid level method is preferable.

In the case of producing a dental printed article using the constrained liquid level method, the lower surface of the vat has light transmittance, and light emitted from below the vat passes through the lower surface of the vat and irradiates the three-dimensional printing composition.

In the case of producing a dental printed article using the three-dimensional printing composition according to the present embodiment, the light irradiating the three-dimensional printing composition is, for example, ultraviolet light having a wavelength of 380 nm to 450 nm, visible light, and the like.

Examples of the light source for irradiating the three-dimensional printing composition include an LED laser, an LED lamp, an LED projector, and the like.

The method of producing the dental printed article may further include a step of cleaning the dental printed article, a step of post-polymerizing the three-dimensional printed article, and the like.

In the method of producing the dental printed article according to the present embodiment, by using the three-dimensional printing composition according to the present embodiment for the three-dimensional printing of the dental printed article, the effect obtained by the three-dimensional printing composition according to the present embodiment can be obtained as well in the obtained dental printed article.

Specifically, as described above, the three-dimensional printing composition according to the present embodiment includes the polymerizable monomer, the inorganic particles, and the photopolymerization initiator, and the inorganic particles are surface-treated with the compound represented by the above general formula (1). Therefore, in the method of producing the dental printed article according to the present embodiment, by the three-dimensional printing using such a three-dimensional printing composition, the obtained dental printed article can exhibit high abrasion resistance and mechanical strength.

Further, as described above, in the three-dimensional printing composition according to the present embodiment, the volume median particle diameter of the inorganic particles is 0.1 µm or more and 2.0 µm or less. Therefore, in the method of producing the dental printed article according to the present embodiment, by the three-dimensional printing using such a three-dimensional printing composition, the obtained dental printed article can maintain high abrasion resistance and mechanical strength.

Further, as described above, the viscosity of the three-dimensional printing composition according to the present embodiment is 200 mPa·s or more and 1500 mPa·s or less. Therefore, in the method of producing the dental printed article according to the present embodiment, by the three-dimensional printing using such a three-dimensional printing composition, the dental printed article obtained by the three-dimensional printing can be easily obtained by the three-dimensional printing.

### Examples

Hereinafter, examples of the present invention will be described, but the present invention is not limited to the examples. In the following, numerical values without units are based on mass (mass%) unless otherwise specified.

### <Production of Inorganic Particles>

Inorganic particles were produced under the conditions presented in Table 1.

### (Inorganic Particles A-1)

Barium glass particles of irregular shape (manufactured by Schott, G018-053) having a volume median particle diameter (hereinafter referred to as an average particle diameter) of 0.4 µm were surface-treated with 8-methacryloyloxyoctyltrimethoxysilane, and inorganic particles A-1 having an average particle diameter of 0.4 µm were obtained.

### (Inorganic Particles A-2)

Except that barium glass particles of irregular shape (manufactured by Schott, G018-053) having an average particle diameter of 0.7 µm were used, inorganic particles A-2 having an average particle diameter of 0.7 µm were obtained in the same manner as the inorganic particles A-1.

### (Inorganic Particles A-3)

Except that barium glass particles of irregular shape (manufactured by Schott, G018-053) having an average particle diameter of 1.5 µm were used, inorganic particles A-3 having an average particle diameter of 1.5 µm were obtained in the same manner as the inorganic particles A-1.

### (Inorganic Particles B-1)

Except that 3-methacryloyloxypropyltrimethoxysilane was used instead of 8-methacryloyloxyoctyltrimethoxysilane, inorganic particles B-1 having an average particle diameter of 0.7 µm were obtained in the same manner as the inorganic particles A-2.

### (Inorganic Particles B-2)

Except that 3-methacryloyloxypropyltrimethoxysilane was used instead of 8-methacryloyloxyoctyltrimethoxysilane, inorganic particles B-2 having an average particle diameter of 1.5 µm were obtained in the same manner as the inorganic particles A-3.

**[Table 1]**

| INORGANIC PARTICLES | AVERAGE PARTICLE DIAMETER | SURFACE TREATMENT AGENT |
|---|---|---|
| A-1 | 0.4 | 8-METHACRYLOYLOXYOCTYLTRIMETHOXYSILANE |
| A-2 | 0.7 | 8-METHACRYLOYLOXYOCTYLTRIMETHOXYSILANE |
| A-3 | 1.5 | 8-METHACRYLOYLOXYOCTYLTRIMETHOXYSILANE |
| B-1 | 0.7 | 3-METHACRYLOYLOXYPROPYLTRIMETHOXYSILANE |
| B-2 | 1.5 | 3-METHACRYLOYLOXYPROPYLTRIMETHOXYSILANE |

### <Preparation of Three-Dimensional Printing composition>

Three-dimensional printing compositions were prepared with the compositions presented in Table 2. The prepared three-dimensional printing compositions were in the form of a paste.

**[Table 2]**

| COMPOSITION | | COMPOUND | EXAMPLE | | | | | | | COMPARATIVE EXAMPLE | | | |
|---|---|---|---|---|---|---|---|---|---|---|---|---|---|
| | | | 1 | 2 | 3 | 4 | 5 | 6 | 7 | 1 | 2 | 3 | 4 |
| | POLYMERIZABLE MONOMER | Bis-MEPP 1 | 30 | 21 | 31 | 62 | 15 | 28 | 34 | 61 | 62 | 46 | 42 |
| | | Bis-MEPP 2 | | 12 | 16 | | 28 | 15 | 9 | | 9 | 10 | |
| | | TEGDMA | 20 | 22 | 6 | 7 | 9 | 5 | 7 | 7 | 7 | 7 | 20 |
| | | UDMA | 8 | 5 | | | | | | | | | 16 |
| | INORGANIC PARTICLES | A-1 | 40 | 38 | | | | | | | | | |
| | | A-2 | | | 45 | 29 | 46 | 50 | | | | | |
| | | A-3 | | | | | | | 48 | | | | |
| CON-DI-TION | | B-1 | | | | | | | | 30 | 20 | | 20 |
| | | B-2 | | | | | | | | | | 35 | |
| | PHOTOPOLYMERIZATION INITIATOR | TPO | 0.5 | 0.5 | 0.5 | 0.5 | 0.5 | 0.5 | 0.5 | 0.5 | 0.5 | 0.5 | 0.5 |
| | TERTIARY AMINE | DME | 0.5 | 0.5 | 0.5 | 0.5 | 0.5 | 0.5 | 0.5 | 0.5 | 0.5 | 0.5 | 0.5 |
| | POLYMERIZATION INHIBITOR | BHT | 0.1 | 0.1 | 0.1 | 0.1 | 0.1 | 0.1 | 0.1 | 0.1 | 0.1 | 0.1 | 0.1 |
| | ULTRAVIOLET ABSORBER | TinuvinP | 0.4 | 0.4 | 0.4 | 0.4 | 0.4 | 0.4 | 0.4 | 0.4 | 0.4 | 0.4 | 0.4 |
| | PIGMENT | | 0.5 | 0.5 | 0.5 | 0.5 | 0.5 | 0.5 | 0.5 | 0.5 | 0.5 | 0.5 | 0.5 |
| | TOTAL | | 100 | 100 | 100 | 100 | 100 | 100 | 100 | 100 | 100 | 100 | 100 |
| E-VAL-UA-TION | VISCOSITY OF PASTE [mPa·s] | | 945 | 903 | 634 | 498 | 957 | 936 | 766 | 1698 | 854 | 982 | 936 |
| | THREE-POINT BENDING STRENGTH [MPa] | | 140 | 132 | 133 | 128 | 140 | 136 | 130 | UNABLE TO PRINT | 109 | 120 | 117 |
| | ABRASION LOSS [*µ* m] | | 4 | 4 | 5 | 7 | 4 | 5 | 18 | UNABLE TO PRINT | 27 | 25 | 20 |

The abbreviations in Table 2 are as follows.

Bis-MEPP 1: Ethoxylated bisphenol A dimethacrylate BPE-100 (manufactured by Shin-Nakamura Chemical Co., Ltd.)
Bis-MEPP 2: Ethoxylated bisphenol A-dimethacrylate BPE-500 (manufactured by Shin-Nakamura Chemical Co., Ltd.)
TEGDMA: Triethylene glycol dimethacrylate
UDMA: Bis(2-methacryloyloxyethyl)-2,2,4-trimethylhexamethylene dicarbamate
TPO: (2,4,6-trimethylbenzoyl)diphenylphosphine oxide
DME: Ethyl 4-dimethylaminobenzoate
BHT: 2,6-di-tert-butyl-p-cresol Tinuvin (registered trademark) P: 2-(2H-benzotriazole-2-yl)-4-methylphenol (manufactured by BASF)
Pigment: Pigment containing titanium dioxide (white)

### <Viscosity of Paste>

The viscosity of the three-dimensional printing composition (paste) was measured at 35°C using a type B viscometer.

The viscosity was evaluated as acceptable (good) when the viscosity was 1,000 mPa·s or less, and as unacceptable (poor) when the viscosity exceeded 1,000 mPa·s.

### <Three-Point Bending Strength>

After designing a 2×2×25 mm test piece using CAD software (Composer (registered trademark), manufactured by Asiga), the test piece was obtained by three-dimensional printing using a 3D printer employing the DLP method (manufactured by Asiga, MAX385) and the three-dimensional printing composition (paste). After thoroughly washing the obtained three-dimensional printed article with isopropanol, the three-dimensional printed article was post-polymerized using a dental photopolymerizer, and the test piece was prepared.

After polishing the test piece using water-resistant abrasive paper, the test piece was stored in water at 37°C for 24 hours. Next, the test piece was subjected to a three-point bending test at a crosshead speed of 1 mm/min using a compact desktop tester (manufactured by Shimadzu Corporation, EZ test), and the three-point bending strength was measured, as the mechanical strength.

The three-point bending strength was evaluated as acceptable (good) when the three-point bending strength was 120 MPa or more, and as unacceptable (poor) when the three-point bending strength was less than 120 MPa.

The prepared three-dimensional printing composition was evaluated by the following tests. The evaluation results are presented in Table 2.

### <Abrasion Loss>

After designing a shape of a test piece using CAD software (Composer (registered trademark), manufactured by Asiga), the test piece was obtained by three-dimensional printing using a 3D printer employing the DLP method (manufactured by Asiga, MAX385) and a paste for three-dimensional printing. After thoroughly washing the three-dimensional printed article with isopropanol, the three-dimensional printed article was post-polymerized using a dental photopolymerizer. The three-dimensional printed article was stored in distilled water at 37°C for 24 hours, and the test piece was obtained.

The test piece was mounted on an attrition and abrasion tester (manufactured by Tokyo Giken Inc.). After polishing an unpolymerized layer with #1000 abrasive paper, the total length of the test piece before the test was measured. A slurry obtained by kneading equal amounts of glycerin and polymethylacrylate (PMMA) (Acrycon AC, manufactured by Mitsubishi Rayon Co., Ltd.) was laid on the attrition and abrasion tester, and a test assuming 100,000 vertical and lateral occlusions against a polymethylacrylate (PMMA) plate was performed. After the test, the full length of the test piece was measured, and the difference between before and after the test was defined as the abrasion loss, to evaluate the abrasion resistance.

The abrasion resistance was evaluated as acceptable (good) when the abrasion loss was 20 µm or less, and as unacceptable (poor) when the abrasion loss exceeded 20 µm.

From Table 2, the three-dimensional printed articles obtained by using the three-dimensional printing composition that includes the polymerizable monomer, the inorganic particles, and the photopolymerization initiator, in which the inorganic particles were surface-treated with 8-methacryloyloxyoctyltrimethoxysilane, exhibited good viscosity, three-point bending strength, and abrasion resistance (Examples 1 to 7).

In contrast, the printed articles obtained by the three-dimensional printing using the three-dimensional printing composition that includes the polymerizable monomer, the inorganic particles, and the photopolymerization initiator, in which the inorganic particles were surface-treated with 3-methacryloyloxypropyltrimethoxysilane, were poor in at least one of viscosity, three-point bending strength, and abrasion resistance (Comparative Examples 1 to 4).

From these results, it was found that the three-dimensional printing composition that includes the polymerizable monomer, the inorganic particles, and the photopolymerization initiator, in which the inorganic particles are surface-treated with the compound represented by the above general formula (1), can improve abrasion resistance and mechanical strength of the obtained three-dimensional printed article while enabling the three-dimensional printing.

Although embodiments of the present invention have been described above, the present invention is not limited to any particular embodiment, and various modifications and alterations are possible within the scope of the invention.

The embodiments disclosed above include, for example, the following aspects.

### (Appendix 1)

A three-dimensional printing composition including:
a polymerizable monomer;
inorganic particles; and
a photopolymerization initiator, wherein
the inorganic particles are surface-treated with a compound represented by a general formula (1) below. 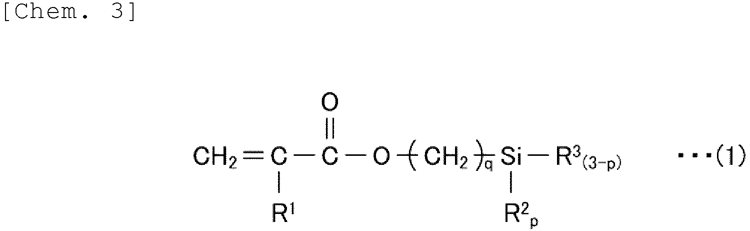
(in the general formula, R1 is a hydrogen atom or a methyl group; R2 is a hydrolyzable group; R3 is a hydrocarbon group having 1 or more and 6 or less carbon atoms; p is 2 or 3; and q is an integer of 5 or more and 13 or less).

### (Appendix 2)

The three-dimensional printing composition of Appendix 1, wherein the volume median particle diameter of the inorganic particles is 0.1 µm or more and 2.0 µm or less.

### (Appendix 3)

The three-dimensional printing composition of Appendix 1 or 2, wherein a viscosity of the three-dimensional printing composition is 200 mPa·s or more and 1,500 mPa·s or less.

### (Appendix 4)

The three-dimensional printing composition according to any one of Appendices 1 to 3, wherein the three-dimensional printing composition is used for three-dimensional printing of a dental printed article.

### (Appendix 5)

A method of producing a dental printed article including:
a step of laminating the three-dimensional printing composition of any one of Appendices 1 to 4, thereby three-dimensionally printing a dental printed article.

The present application is based on and claims priority to Japanese Patent Application No. 2022-099509, filed June 21, 2022, the contents of which are incorporated herein by reference in their entirety.

## Claims

1. A three-dimensional printing composition comprising:
a polymerizable monomer;
inorganic particles; and
a photopolymerization initiator, wherein
the inorganic particles are surface-treated with a compound represented by a general formula (1) 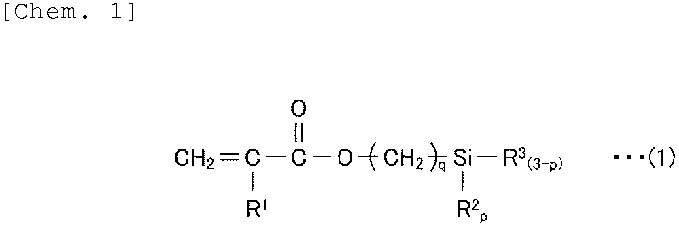
(in the general formula, R1 is a hydrogen atom or a methyl group; R2 is a hydrolyzable group; R3 is a hydrocarbon group having 1 or more and 6 or less carbon atoms; p is 2 or 3; and q is an integer of 5 or more and 13 or less).

2. The three-dimensional printing composition according to claim 1, wherein a volume median particle diameter of the inorganic particles is 0.1 µm or more and 2.0 µm or less.

3. The three-dimensional printing composition according to claim 1, wherein a viscosity of the three-dimensional printing composition is 200 mPa·s or more and 1,500 mPa·s or less.

4. The three-dimensional printing composition according to any one of claims 1 to 3, wherein the three-dimensional printing composition is used for three-dimensional printing of a dental printed article.

5. A method of producing a dental printed article comprising:
a step of laminating the three-dimensional printing composition of any one of claims 1 to 3, thereby three-dimensionally printing a dental printed article.
